# FASCICULE DE BREVET EUROPEEN

(11) **EP 2 486 020 B1**
(45) Date de publication et mention de la délivrance du brevet: **16.08.2017**
(21) Numéro de dépôt: 10763362.0
(22) Date de dépôt: 06.10.2010
(51) Int. Cl.: C07D 233/84

(54) **PROCEDE DE SYNTHESE DE L'ERGOTHIONEINE ET ANALOGUES**
VERFAHREN ZUR SYNTHESE VON ERGOTHIONEIN UND DERGLEICHEN
METHOD FOR THE SYNTHESIS OF ERGOTHIONEINE AND THE LIKE

(30) Priorité: 06.10.2009 FR 0956962
(43) Date de publication de la demande: 15.08.2012
(73) Titulaire: Tetrahedron, 75001 Paris (FR)
(72) Inventeur: ERDELMEIER, Irene, F-75013 Paris (FR)
(74) Mandataire: Regimbeau
(86) Numéro de dépôt international: PCT/EP2010/064950
(87) Numéro de publication internationale: WO 2011/042480

(56) Documents cités:
- US-A1- 2009 093 642
- ISHIKAWA Y ET AL: "Participation of an intermediate sulfoxide in the enzymatic thiolation of the imidazole ring of hercynine to form ergothioneine.", THE JOURNAL OF BIOLOGICAL CHEMISTRY 25 JUL 1974, vol. 249, no. 14, 25 juillet 1974 (1974-07-25), pages 4420-4427, XP002568730, ISSN: 0021-9258 cité dans la demande
- XU J ET AL: "Synthesis of L-(+)-ergothioneine", JOURNAL OF ORGANIC CHEMISTRY 1995 US, vol. 60, no. 20, 1995, pages 6296-6301, XP002568731, ISSN: 0022-3263 cité dans la demande
- SHOSUKE ITO: "Synthesis of 2-S-cysteinylhistidine and 2-mercaptohistidine via bromolactone derivative of histidine", JOURNAL OF ORGANIC CHEMISTRY, vol. 50, no. 19, septembre 1985 (1985-09), pages 3636-3638, XP009129621, DOI: 10.1021/jo00219a044

## Description

La présente demande de brevet concerne un nouveau procédé de synthèse de l'ergothonéine et de dérivés apparentés.

L'Ergothionéine, découverte en 1909 par Tanret dans l'ergot de seigle, est un acide aminé d'origine naturelle, aux propriétés antioxydantes, répondant à la formule suivante :

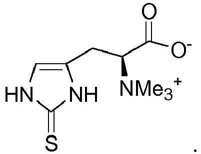

Sa présence a été démontrée non seulement dans un grand nombre de champignons et de mycobactéries, mais aussi chez les végétaux, les animaux et l'Homme. C'est au niveau de leurs racines que les végétaux absorbent l'ergothionéine biosynthétisée par les champignons qui y sont fixés. Ce n'est qu'au travers de leur alimentation que les organismes supérieurs, et l'Homme en particulier, ingèrent ce composé.

Plusieurs synthèses de cette molécule ont ainsi été proposées dans la littérature, deux d'entre elles seulement permettant d'aboutir à la L-ergothionéine (énantiomère naturel).

La demande internationale WO 95/00 494 propose une synthèse en 5 étapes de la L-ergothionéine par réaction de l'ester méthylique de la Nα,Nα-diméthyl-histidine (elle-même obtenue à partir de L-histidine en 2 étapes) avec du chlorothioformiate de phényle, puis réaction avec du chloroformiate d'éthyle, formation de l'ammonium quaternaire et enfin déprotection du soufre et de l'ester méthylique. Ainsi, une telle stratégie de synthèse nécessite la protection du soufre, qui ne peut être présent sous forme libre, afin de permettre la méthylation du groupement diméthylamine pour conduire à la fonction bétaïne. De plus, le chlorothioformiate de phényle doit être préparé à partir du thiophosgène (CSCl₂), réactif toxique et difficilement disponible en grande quantité pour une utilisation à l'échelle industrielle.

La demande de brevet US 2009/093 642 décrit également une synthèse en 9 étapes de la L-ergothionéine à partir de l'histidine par ouverture du cycle imidazole et réaction avec un thiocyanate tel que le thiocyanate de potassium, pour donner la 2-thiohistidine (selon la méthode décrite par Heath, H. et al., 1951, J.Chem.Soc., 2215), puis protection du soufre par un groupement tertiobutyle, formation de l'ammonium quaternaire et déprotection du soufre. Outre l'utilisation de grands volumes d'acide chlorhydrique, le KSCN, utilisé en milieu acide, est un réactif hautement toxique.

Ces deux procédés présentent plusieurs points communs. Outre leur nombre d'étapes élevé, ils cumulent les inconvénients d'utiliser non seulement des réactifs très toxiques mais aussi des quantités importantes de solvants organiques et d'acide chlorhydrique concentré, d'où des risques sur le plan environnemental.

Sur le plan de la stratégie de synthèse, ces deux procédés ont en commun d'introduire le soufre, à partir de l'histidine ou de l'un de ses dérivés N-diméthylés, avant de générer le groupement bétaïne, ce qui présente l'inconvénient d'alourdir la synthèse par des étapes supplémentaires de protection et de déprotection.

Ishikawa et al. (the Journal of Biological Chemistry 1974, vol 249, No 14, Issue of July 25, pp 4420-4427) décrivent également un procédé enzymatique d'obtention de l'ergothionéine à partir de l'hercynine.

Ainsi, il existe un réel besoin de développer un nouveau procédé de synthèse de l'ergothionéine et de ses dérivés qui soit applicable au niveau industriel, c'est-à-dire qui ne présente pas de difficultés de purification, qui n'utilise pas de produits ou de solvants dangereux et toxiques pour l'Homme et l'environnement, et qui permet d'accéder, à l'échelle industrielle, au produit avec un bon rendement et un faible coût.

Dans le souci de développer un procédé respectueux de l'environnement, en utilisant à la fois des réactifs peu toxiques, en minimisant le nombre d'étapes ainsi qu'en réalisant des réactions en milieu aqueux, la Demanderesse a décidé d'opter pour une stratégie de synthèse « inversée » qui consiste à introduire le soufre sur un intermédiaire possédant déjà le groupement bétaïne. Cette approche, totalement originale, peut être qualifiée de bio-mimétique dans la mesure où la biosynthèse par voie enzymatique de la L-ergothionéine dans les champignons procède de la même façon (Askari, A. et Melville, D.B., 1962, J. Biol. Chem., 237, 1615-1618).

La présente demande de brevet a ainsi pour objet un procédé de synthèse d'un dérivé de formule (I) suivante : 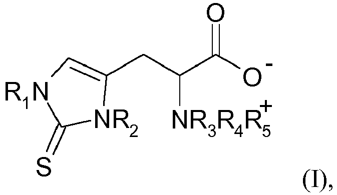 ou d'un sel physiologiquement acceptable de celui-ci, d'un tautomère, d'un stéréoisomère ou d'un mélange de stéréoisomères en toutes proportions, en particulier d'un mélange d'énantiomères, et notamment d'un mélange racémique de celui-ci, pour laquelle :
- R₁ et R₂ représentent, indépendamment l'un de l'autre, un atome d'hydrogène ou un groupe (C₁-C₄)alkyle tel que méthyle, au moins un des groupements R₁ et R₂ représentant un atome d'hydrogène, et avantageusement représentant chacun un atome d'hydrogène, et
- R₃, R₄ et R₅ représentent, indépendamment les uns des autres, un groupe (C₁-C₄)alkyle tel que méthyle,
comprenant les étapes successives suivantes :
(i) réaction de clivage d'un composé de formule (II) suivante : 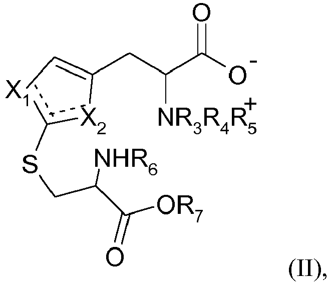 ou d'un sel physiologiquement acceptable de celui-ci, d'un tautomère, d'un stéréoisomère ou d'un mélange de stéréoisomères en toutes proportions, en particulier d'un mélange d'énantiomères, et notamment d'un mélange racémique de celui-ci,
   pour laquelle :
   - 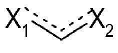 represente 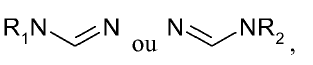
   - R₁, R₂, R₃, R₄ et R₅ sont tels que définis ci-dessus,
   - R₆ représente un atome d'hydrogène ou un groupe (C₁-C₄)alkyle ou -CO-((C₁-C₄)alkyle), et en particulier un atome d'hydrogène ou un groupe -COCH₃, et plus particulièrement un atome d'hydrogène, et
   - R₇ représente un atome d'hydrogène ou un groupe (C₁-C₄)alkyle, et en particulier un atome d'hydrogène,
   en présence d'un thiol, de préférence soluble dans le solvant de réaction qui pourra être notamment de l'eau, à une température supérieure ou égale à 60°C, pour donner un composé de formule (I), et
(ii) séparation du composé de formule (I) obtenu à l'étape (i) précédente du milieu réactionnel.

Par « tautomère », on entend, au sens de la présente invention, un isomère de constitution du composé obtenu par prototropie, c'est-à-dire par migration d'un atome d'hydrogène et changement de localisation d'une double liaison. Les différents tautomères d'un composé sont généralement interconvertibles et présents en équilibre, en solution, dans des proportions qui peuvent varier selon le solvant utilisé, la température ou encore le pH.

Dans le cadre des composés de l'invention, le cycle 2-thioimidazole peut être présent sous les différentes formes tautomères suivantes :

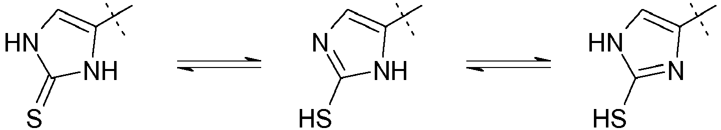

Dans la présente invention, on entend désigner par « physiologiquement acceptable » ce qui est généralement sûr, non toxique et ni biologiquement ni autrement non souhaitable et qui est acceptable pour une utilisation pharmaceutique, cosmétique ou alimentaire (humaine ou animale), en particulier alimentaire.

On entend désigner par « sels physiologiquement acceptables » d'un composé, des sels qui sont physiologiquement acceptables, comme défini ci-dessus, et qui possèdent l'activité (pharmacologique, cosmétique ou alimentaire) souhaitée du composé parent. De tels sels comprennent :
(1) les hydrates et les solvates,
(2) les sels d'addition d'acide formés avec des acides inorganiques tels que l'acide chlorhydrique, l'acide bromhydrique, l'acide sulfurique, l'acide nitrique, l'acide phosphorique et similaires ; ou formés avec des acides organiques tels que l'acide acétique, l'acide benzènesulfonique, l'acide benzoïque, l'acide camphresulfonique, l'acide citrique, l'acide éthanesulfonique, l'acide fumarique, l'acide glucoheptonique, l'acide gluconique, l'acide glutamique, l'acide glycolique, l'acide hydroxynaphtoïque, l'acide 2-hydroxyéthanesulfonique, l'acide lactique, l'acide maléique, l'acide malique, l'acide mandélique, l'acide méthanesulfonique, l'acide muconique, l'acide 2-naphtalènesulfonique, l'acide propionique, l'acide salicylique, l'acide succinique, l'acide dibenzoyl-L-tartrique, l'acide tartrique, l'acide p-toluènesulfonique, l'acide triméthylacétique, l'acide trifluoroacétique et similaires, ou
(3) les sels formés lorsqu'un proton acide présent dans le composé parent est soit remplacé par un ion métallique, par exemple un ion de métal alcalin, un ion de métal alcalino-terreux ou un ion d'aluminium ; soit coordonné avec une base organique ou inorganique. Les bases organiques acceptables comprennent la diéthanolamine, l'éthanolamine, N-méthylglucamine, la triéthanolamine, la trométhamine et similaires. Les bases inorganiques acceptables comprennent l'hydroxyde d'aluminium, l'hydroxyde de calcium, l'hydroxyde de potassium, le carbonate de sodium et l'hydroxyde de sodium.

Par « stéréoisomères », on entend, au sens de la présente invention, des diastéréoisomères et des énantiomères. Il s'agit donc d'isomères optiques. Les stéréoisomères qui ne sont pas des images dans un miroir l'un de l'autre sont désignés par « diastéréoisomères », et les stéréoisomères qui sont des images dans un miroir l'un de l'autre, mais non superposables, sont désignés par « énantiomères ».

Un mélange contenant des quantités égales de deux formes énantiomères individuelles de chiralité opposée est désigné par « mélange racémique ».

Par groupement « (C₁-C₄)alkyle », on entend, au sens de la présente invention, une chaîne hydrocarbonée saturée, linéaire ou ramifiée, comportant 1 à 4 atomes de carbone. Il pourra s'agir des groupes méthyle, éthyle, propyle, isopropyle, butyle, isobutyle, sec-butyle, ou tert-butyle. En particulier, il s'agira du groupe méthyle.

Par « thiol », on entend, au sens de la présente invention, tout réactif contenant un groupement SH dans sa structure moléculaire. Il s'agira plus particulièrement d'un composé de formule R-SH avec R représentant une chaine hydrocarbonée saturée en C₁ à C₈, notamment en C₂ à C₆, linéaire ou ramifiée, substituée par un ou plusieurs substituants polaires.

Par « chaîne hydrocarbonée saturée », on entend, au sens de la présente invention, une chaîne hydrocarbonée saturée, linéaire ou ramifiée, comprenant avantageusement 1 à 8 atomes de carbone. Il pourra s'agir plus particulièrement d'une chaîne saturée, linéaire, telle qu'un groupe méthyle, éthyle, propyle, butyle, pentyle ou encore hexyle.

Par substituants polaires, on entend, au sens de la présente invention, des groupements hydrophiles tels que les groupements OH, SH, NH₂ et COOH.

Par « réaction de clivage », on entend, au sens de la présente invention, que le composé engagé dans cette réaction est scindé en deux parties lors de cette réaction, pour permettre dans le cas présent de former la fonction thiocarbonyle du composé de formule (I).

Le composé de formule (I) pourra être en particulier un composé de formule (Ia) suivante : 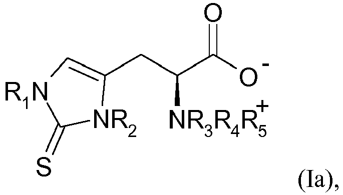 ou un sel physiologiquement acceptable, un tautomère, un stéréoisomère ou un mélange de stéréoisomères en toutes proportions, en particulier un mélange d'énantiomères, et notamment un mélange racémique de celui-ci,
pour laquelle R₁, R₂, R₃, R₄ et R₅ sont tels que définis précédent.

Le composé de formule (I) représentera notamment l'ergothionéine, et en particulier la L-ergothionéine.

### Etape (i) :

Cette réaction de clivage, réalisée en présence d'un thiol, permet d'obtenir le composé de formule (I) ainsi que de l'acide pyruvique (CH₃C(O)-CO₂H) ou un de ses dérivés, notamment un ester (CH₃C(O)-CO₂R₇) ou un dérivé obtenu par réaction avec le thiol, tel qu'un dérivé thiocétalique (deux molécules de thiol pouvant réagir avec la fonction cétone de l'acide pyruvique).

Par ailleurs, le thiol devra être de préférence soluble dans le solvant de réaction qui pourra être notamment de l'eau, ce qui présente l'avantage supplémentaire d'être plus écologique.

Le thiol utilisé dans cette étape (i) pourra être plus particulièrement un thiol répondant à la formule R-SH, avec R représentant une chaîne alkyle, linéaire ou ramifiée, et de préférence linéaire, comportant de 1 à 8, notamment 2 à 6, en particulier 2 à 4, atomes de carbone, substituée par un ou plusieurs groupes choisis parmi OH, SH, NH₂ et COOH.

La présence de groupements hydrophiles (OH, SH, NH₂ et COOH) pourra permettre notamment de rendre le thiol plus soluble dans l'eau, lorsque l'eau est utilisée comme solvant.

Le thiol pourra plus particulièrement être choisi parmi la cystéine, le dithiothréitol, le 2-mercaptoéthanol, l'acide 2-mercaptopropionique, l'acide 3-mercaptopropionique et l'acide thioglycolique, et de préférence sera l'acide 3-mercaptopropionique.

Il pourra s'agir également de l'acide mercaptoacétique et l'acide mercaptohexanoïque.

Avantageusement, on utilisera au moins 2 équivalents molaires de thiol par rapport au composé (II), c'est-à-dire qu'au moins 2 moles de thiol sont utilisées pour une mole de composé (II) utilisée. On pourra en particulier utiliser au moins 5 équivalents molaires de thiol, et notamment 5 à 10 équivalents molaires de thiol par rapport au composé (II).

Le mélange réactionnel est chauffé à une température supérieure à 60°C car en-dessous de cette température, la cinétique de la réaction serait trop lente. La réaction pourra être réalisée à une température comprise entre 60 et 120°C, avantageusement entre 80 et 100°C, plus particulièrement après addition du thiol.

La réaction pourra être réalisée notamment en milieu acide.

### Etape (ii) :

Le produit final obtenu (composé de formule (I)) pourra être séparé du milieu réactionnel par des techniques bien connues de l'homme du métier et applicables à l'échelle industrielle, en particulier par évaporation, éventuellement partielle, des solvants, suivie de préférence d'une recristallisation pour purifier le produit.

Les composés de formule (I) étant solubles dans l'eau, une ou plusieurs extractions préalables avec un solvant organique, tel que par exemple l'acétate d'éthyle ou l'éther tert-butyl-méthylique, pourront permettre d'éliminer les sous-produits organiques formés au cours de la réaction, tels que l'acide pyruvique ou ses dérivés, ainsi que l'excès de thiol.

Le produit obtenu pourra être purifié si nécessaire par des techniques bien connues de l'homme du métier, par exemple par recristallisation, éventuellement après désalinisation de la phase aqueuse le contenant, par des techniques bien connues de l'homme du métier (par exemple par électrodialyse, par ajout d'une résine adéquate, ou par osmose inverse).

Avant ou après cette étape (ii), un sel du composé formé pourra être préparé, si cela est souhaité, notamment par addition d'un acide ou d'une base physiologiquement acceptable tel que défini précédemment.

Le composé de formule (II) pourra être préparé à partir d'un sel d'addition d'acide, à l'exclusion du sel de l'acide iodhydrique (HI), d'un composé de type bétaïne de formule (III) suivante : 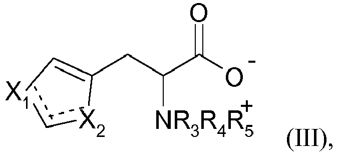 ou d'un tautomère, d'un stéréoisomère ou d'un mélange de stéréoisomères en toutes proportions, en particulier d'un mélange d'énantiomères, et notamment d'un mélange racémique de celui-ci,
pour laquelle 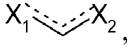 R₃, R₄ et R₅ sont tels que définis précédemment, par réaction successivement avec du dibrome,
puis avec un dérivé de cystéine de formule (IV) suivante : 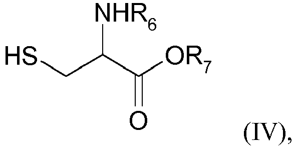 ou un stéréoisomère ou un mélange de stéréoisomères en toutes proportions, en particulier un mélange d'énantiomères, et notamment un mélange racémique de celui-ci, dans laquelle R₆ et R₇ sont tels que définis précédemment.

Par « sel d'addition d'acide du composé de type bétaïne de formule (III) », on entend, au sens de la présente invention, un sel du composé de type bétaïne de formule (III) obtenu par addition d'un acide, à l'exclusion de l'acide iodhydrique HI. L'acide pourra être en particulier de l'acide chlorhydrique ou de l'acide sulfurique.

Dans cette réaction, le dibrome pourra être utilisé à raison de 1 à 1,5 équivalent molaire par rapport au composé de type bétaïne de formule (III).

De préférence, le dibrome est ajouté à froid (addition très rapide de préférence), à une température inférieure à 10°C, de préférence inférieure à 5°C. L'addition du dibrome pourra donc être réalisée à une température comprise entre -10°C et 10°C, avantageusement comprise entre -5°C et 5°C.

Le dérivé de cystéine pourra être en particulier la N-acétylcystéine ou la cystéine (notamment sous forme D, L ou racémique), et en particulier de la cystéine et notamment de la L-cystéine.

Le dérivé de cystéine sera avantageusement utilisé en excès, en particulier à raison de 2 à 10, avantageusement 3 à 7 équivalents molaires de dérivé de cystéine par rapport au composé de type bétaïne de formule (III), c'est-à-dire que 2 à 10, avantageusement 3 à 7 moles de dérivé de cystéine sont utilisées pour une mole de composé (III) utilisée.

Cette réaction pourra être réalisée dans un solvant tel que l'eau.

Le rendement de cette étape pourra être supérieur ou égal à 45%, voire supérieur ou égal à 60%.

De préférence, le composé de formule (II) ne sera pas isolé du milieu réactionnel mais sera engagé directement dans l'étape (i) suivante. Ainsi, la préparation du composé (I) à partir du composé (III) peut être réalisée dans un seul réacteur, sans isolement du composé (II) intermédiaire (réaction « one-pot »).

Le procédé de préparation d'un composé de formule (I) selon l'invention pourra donc comprendre les étapes successives suivantes :
(**a1**) réaction d'un sel d'addition d'acide, à l'exclusion du sel de l'acide iodhydrique, d'un composé de type bétaïne de formule (III) tel que défini ci-dessus, ou d'un tautomère, d'un stéréoisomère ou d'un mélange de stéréoisomères en toutes proportions, en particulier d'un mélange d'énantiomères, et notamment d'un mélange racémique de celui-ci,
   avec du dibrome,
   puis avec un dérivé de cystéine de formule (IV) tel que défini ci-dessus ou un stéréoisomère ou un mélange de stéréoisomères en toutes proportions, en particulier un mélange d'énantiomères, et notamment un mélange racémique de celui-ci, et en particulier avec de la cystéine et notamment de la L-cystéine,
   pour donner une composé de formule (II) tel que défini ci-dessus,
**(b1)** réaction de clivage du composé de formule (II) obtenu à l'étape (a1) précédente en présence d'un thiol tel que défini précédemment, de préférence soluble dans le solvant de réaction qui pourra être notamment de l'eau, à une température supérieure ou égale à 60°C, pour donner un composé de formule (I), et
(**c1**) séparation du composé de formule (I) obtenu à l'étape (b1) précédente du milieu réactionnel.

Les étapes (b1) et (c1) correspondent respectivement aux étapes (i) et (ii) précédentes. L'étape (a1), quant à elle, correspond à l'étape de préparation du composé de type bétaïne de formule (II) décrite précédemment.

Avantageusement, les étapes (a1) et (b1) seront réalisées dans un même solvant, tel que l'eau, de préférence, dans un même réacteur, c'est-à-dire sans isolement des produits intermédiaires (composé de formule (II) en particulier).

Dans ces conditions, le milieu réactionnel pourra contenir un dérivé de cystéine utilisé de préférence en excès à l'étape (a1). Avant de séparer le composé de formule (I) du milieu réactionnel (étape (c1)), il pourra donc être nécessaire d'éliminer l'excès de dérivé de cystéine afin de faciliter l'isolement et la purification du composé de formule **(I)**. Notamment, dans le cas d'un dérivé de cystéine pour lequel R₇ = H ou (C₁-C₄)alkyle et en particulier dans le cas de la cystéine, il peut être ajouté par exemple du benzaldéhyde qui formera alors avec le dérivé de cystéine en excès un dérivé de l'acide 2-phénylthiazolidine-4-carboxylique, composé qui précipite dans un solvant tel que l'eau. Par ce moyen, le dérivé de cystéine en excès pourrait être recyclée.

Le rendement global de préparation du composé de formule (I) à partir du composé de type bétaïne de formule (III) pourra être supérieur ou égal à 40%.

Selon un mode de réalisation particulier de l'invention, le composé de formule (I) est un composé de formule (Ia) et son procédé de préparation comprend les étapes successives suivantes :
**(a2)** réaction d'un sel d'addition d'acide, à l'exclusion du sel de l'acide iodhydrique, d'un composé de type bétaïne de formule (IIIa) suivante : 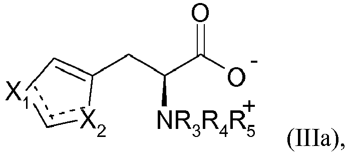 ou d'un tautomère, d'un stéréoisomère ou d'un mélange de stéréoisomères en toutes proportions, en particulier d'un mélange d'énantiomères, et notamment d'un mélange racémique de celui-ci, pour laquelle 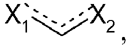 R₃, R₄ et R₅ sont tels que définis précédemment, successivement avec du dibrome,
   puis avec un dérivé de cystéine de formule (IV) tel que défini ci-dessus ou un stéréoisomère ou un mélange de stéréoisomères en toutes proportions, en particulier un mélange d'énantiomères, et notamment un mélange racémique de celui-ci, et en particulier avec de la cystéine et notamment de la L-cystéine,
   pour donner un composé de type bétaïne de formule (IIa) suivante : 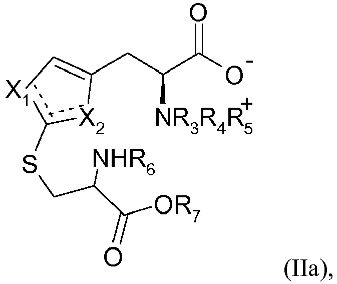 ou un sel physiologiquement acceptable, un tautomère, un stéréoisomère ou un mélange de stéréoisomères en toutes proportions, en particulier un mélange d'énantiomères, et notamment un mélange racémique de celui-ci,
   pour laquelle 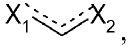 R₃, R₄, R₅, R₆ et R₇ sont tels que définis précédemment,
(b2) réaction de clivage du composé de type bétaïne de formule (IIa) obtenu à l'étape (a2) précédente en présence d'un thiol tel que défini précédemment, de préférence soluble dans le solvant de réaction qui pourra être notamment de l'eau, et en particulier avec la cystéine, le dithiothréitol, le 2-mercaptoéthanol, l'acide 2-mercaptopropionique, l'acide 3-mercaptopropionique ou l'acide thioglycolique, et de préférence avec l'acide 3-mercaptopropionique, à une température supérieure ou égale à 60°C,
   pour donner un composé de formule (Ia), et
**(c2)** séparation du composé de formule (Ia) obtenu à l'étape (b2) précédente du milieu réactionnel.

Les étapes (a2), (b2) et (c2) correspondent respectivement aux étapes (a1), (b1) et (c1) précédentes.

Les composés de formule (IIa) représentent des formes particulières du composé de formule (II). De même, les composés de type bétaïne de formule (IIIa) représentent des formes particulières du composé de type bétaïne de formule (III).

La présente invention a également pour objet l'utilisation d'un composé de formule (II) suivante : 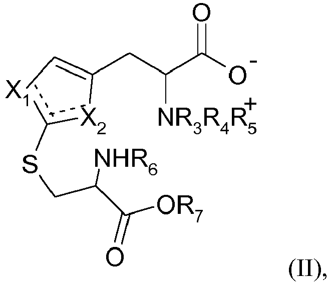 ou un sel physiologiquement acceptable, un tautomère, un stéréoisomère ou un mélange de stéréoisomères en toutes proportions, en particulier un mélange d'énantiomères, et notamment un mélange racémique de celui-ci, pour laquelle :
- 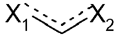 représente 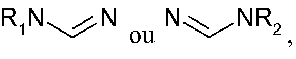 et
- R₁, R₂, R₃, R₄, R₅, R₆ et R₇ sont tels que définis précédemment, à l'exclusion du composé pour lequel 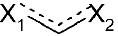 représente 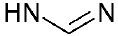 ou 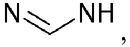 R₃, R₄ et R₅ représentent chacun un groupe méthyle et R₆ et R₇ représentent chacun un atome d'hydrogène
   pour la préparation d'un dérivé de formule (I) suivante : 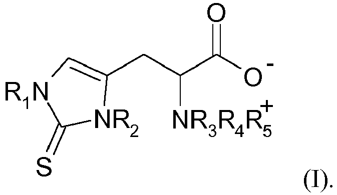 par synthèse chimique comprenant le clivage en présence d'un thiol par chauffage à une température supérieure ou égale à 60°C.

Le composé exclu est décrit dans : Ishikawa et al. J. Biol. Chem. 1974, 249(14), 4420.

En particulier, il ne s'agira pas d'un composé de formule (II) pour lequel 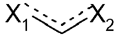 représente 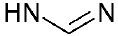 ou 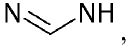 et R₃, R₄ et R₅ représentent chacun un groupe méthyle.

Il pourra s'agir notamment d'un composé de formule (IIa) telle que défini précédemment. En particulier, ce composé pourra être le dichlorhydrate du 2-{2-[(2-ammonio-2-carboxyéthyl)thio]-1*H-*imidazol-4-yl}-1-carboxy-*N*,*N*,*N-*triméthyléthanaminium (Herc-Cys, 2HCl)

La présente invention décrit également un procédé de préparation d'un composé de formule(II) suivante : 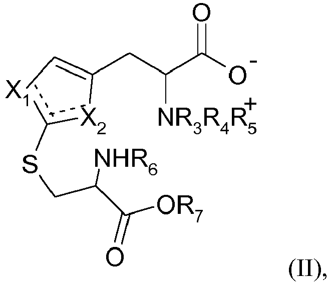 ou un sel physiologiquement acceptable, un tautomère, un stéréoisomère ou un mélange de stéréoisomères en toutes proportions, en particulier un mélange d'énantiomères, et notamment un mélange racémique de celui-ci, pour laquelle :
- 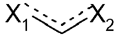 représente 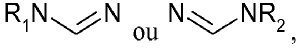 et
- R₁, R₂, R₃, R₄, R₅, R₆ et R₇ sont tels que définis précédemment,
par réaction d'un sel d'addition d'acide, à l'exclusion du sel de l'acide iodhydrique, d'un composé de type bétaïne de formule (III) tel que défini précédemment ou d'un tautomère, d'un stéréoisomère ou d'un mélange de stéréoisomères en toutes proportions, en particulier d'un mélange d'énantiomères, et notamment d'un mélange racémique de celui-ci,
pour laquelle 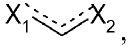 R₃, R₄ et R₅ sont tels que définis précédemment, successivement avec du dibrome, puis avec un dérivé de cystéine de formule (IV) tel que défini précédemment.

Dans cette réaction, le dibrome pourra être utilisé à raison de 1 à 1,5 équivalents molaires par rapport au composé de type bétaïne de formule (III).

De préférence, le dibrome est ajouté à froid (addition très rapide de préférence), à une température inférieure à 10°C, de préférence inférieure à 5°C. L'addition du dibrome pourra donc être réalisée à une température comprise entre -10°C et 10°C, avantageusement comprise entre -5°C et 5°C.

Le dérivé de cystéine pourra être en particulier la N-acétylcystéine ou la cystéine (notamment sous forme D, L ou racémique), et en particulier de la cystéine et notamment de la L-cystéine.

Le dérivé de cystéine sera avantageusement utilisé en excès, en particulier à raison de 2 à 10, avantageusement 3 à 7 équivalents molaires de dérivé de cystéine par rapport au composé de type bétaïne de formule (III), c'est-à-dire que 2 à 10, avantageusement 3 à 7 moles de dérivé de cystéine sont utilisées pour une mole de composé (III) utilisée.

Cette réaction pourra être réalisée dans un solvant tel que l'eau.

La présente invention sera mieux comprise à la lumière des exemples qui suivent, qui sont fournis simplement à titre d'illustration et ne sauraient en aucune façon limiter la portée de l'invention.

### EXEMPLES

Toutes les réactions sont réalisées à l'air libre sauf indication contraire.

### 1- Préparation des composés de formule (II) selon l'invention

### Exemple 1 : Préparation du dichlorhydrate du 2-{2-[(2-ammonio-2-carboxyéthyl)thio]-1H-imidazol-4-yl}-1-carboxy-N,N,N-triméthyléthanaminium (Herc-Cys, 2HCl)

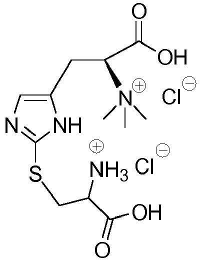

986 mg (5 mmoles) d'Hercynine (V. N. Reinhold et al., J. Med. Chem. 11, 258 (1968*))* sont dissous dans 10 mL d'eau. On ajoute 417 µL (5 mmoles) d'acide chlorhydrique concentré, puis on refroidit la solution à 0°C. Sous très forte agitation, 308 µL (959 mg, 6 mmoles, 1,2 équiv.) de dibrome sont ajoutés goutte à goutte (temps d'addition 1min20). Le mélange réactionnel se colore en jaune, et un solide rougeâtre se forme. Cinq minutes après la fin de l'addition du dibrome, 1,87 g (15 mmoles, 3 équiv.) de L-Cystéine sont ajoutés. Immédiatement, le mélange se décolore, et le précipité rougeâtre se dissout en quelques minutes.

Après agitation à 0°C pendant 1h, le mélange est filtré, et le précipité lavé avec 2x0,5 mL d'eau.

Le filtrat est déposé sur une colonne remplie avec 75g de DOWEX^{®} 50WX2-400, conditionnée auparavant avec de l'acide chlorhydrique HCl 1N. Après élution avec 400 mL d'acide chlorhydrique HCl 1N, puis 500 mL d'acide chlorhydrique HCl 2N, les fractions contenant le produit désiré sont réunies. Après évaporation et 2 co-évaporations avec 2x20 mL de toluène, on obtient après séchage 894 mg (46%) du produit désiré sous forme de cristaux jaunes.

*(Ce produit a été synthétisé avec un faible rendement à partir de l'Ergothionéine et de la Chloroalanine, mais sous forme acide aminé libre, par* Ishikawa et al., J. Biol. Chem. 249 (14), 4420 (1974*).)*
¹H-RMN (D₂O/DCl, 400 MHz) : δ (ppm) = 3,14 (s, 9H) ; 3,37 (m, 2H) ; 3,56 (m, 2H) ; 4,20 (m, 1H) ; 4,28 (m, 1H) ; 7,31 (s, 1H).
UPLC-MS (ES+) : 317,4 (MH+)

### Exemple 2 : Préparation du dichlorhydrate du 2-{2-[(2-ammonio-2-carboxyethyl)thio]-1H-imidazol-4-yl}-1-carboxy-N,N,N-trimethylethanaminium dichloride (Herc-Cys, 2HCl) - variation de la quantité de L-Cystéine

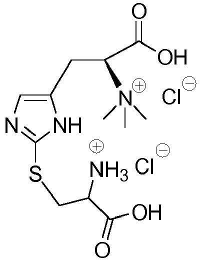

On procède comme décrit dans l'Exemple 1, sauf que l'on ajoute 3,12g (25 mmoles, 5 équiv.) de L-Cystéine. Sept minutes après la fin de l'addition de dibrome.

Après traitement et purification sur colonne de DOWEX^{®}, on obtient après séchage 1,13 g (58%) du produit désiré sous forme de cristaux jaunes.

L'analyse ¹H-RMN (D₂O/DCl) est identique à celle décrite dans l'Exemple 1.

### 2- Préparation des composés de formule (I) selon l'invention à partir des intermédiaires de formule (II)

### Exemple 3 : Préparation de la L-Ergothionéine par clivage de Herc-Cys, 2HCl

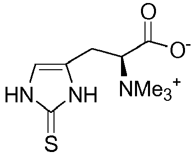

1,67g (4,4 mmoles) de Herc-Cys, 2HCl sont solubilisés dans 16,7 mL d'eau, et on ajoute 1,895 mL (2,29g, 21,39 mmoles, 5 équiv.) d'acide 3-mercaptopropionique. Le mélange limpide, légèrement jaune, est chauffé sous agitation pendant 24h à 85°C. Ensuite on ajoute de nouveau 1,895 mL (2,29g, 21,39 mmoles, 5 équiv.) d'acide 3-mercaptopropionique, et on poursuit le chauffage pendant 48h.

Le mélange réactionnel est refroidit à 0°C, et un précipité blanc se forme. Après filtration et rinçage du précipité avec 2x2 mL d'eau froide, on lave le filtrat avec 5x20 mL de dichlorométhane et 5x20 mL d'acétate d'éthyle.

La phase aqueuse est évaporée sous vide, et le résidu dissout dans 33 mL d'eau. Par addition d'une solution d'ammoniaque à 20%, le pH de la solution est ajusté à 6. Après lyophilisation, la poudre obtenue est solubilisée à chaud dans un mélange éthanol/eau (7/1). Après addition de charbon activé (100 mg) et filtration à chaud sur Clarcel, la solution obtenue est gardée à froid pendant 14h. Après filtration et séchage on obtient 676 mg (69%) de L-Ergothionéine sous forme de poudre blanche.

Les données analytiques obtenues sont identiques à celles obtenues dans la littérature (J. Xu, J. C. Yadan, J. Org. Chem. 60, 6296 - 6301 (1995*)).*
¹H-RMN (D₂O, 400 MHz) : δ (ppm) = 3,20 (m, 2H) ; 3,29 (s, 9H) ; 3,90 (dd, J = 11 Hz, J = 5 Hz, 1 H) ; 6, 81 (s, 1 H).
UPLC-MS (ES+): 230,6 (MH+)

### 3- Préparation des composés de formule (I) selon l'invention sans isolement des intermédiaires de formule (II)

### Exemple 4 : Préparation de la L-Ergothionéine à partir de l'Hercynine (« one-pot ») - purification après désalinisation de la phase aqueuse avec une résine

### a) Formation de l'adduit Herc-Cys (composé de formule (II))

19,72 g (0,1 mole) d'Hercynine (V. N. Reinhold et al., J. Med. Chem. 11, 258 (1968*)*) sont dissous dans 200 mL d'eau. On ajoute 8,35 mL (0,1 mole) d'acide chlorhydrique concentré, puis on refroidit la solution à 2°C. Sous très forte agitation, 6,68 mL (20,77 g, 130 mmoles, 1,3 équiv.) de dibrome sont ajoutés goutte à goutte sans dépasser 3°C (temps d'addition 10min). Le mélange réactionnel se colore en jaune, et un solide rougeâtre se forme. Sept minutes après la fin de l'addition du dibrome, 62,4 g (0,5 moles, 5 équiv.) de L-Cystéine sont ajoutés, et la température interne monte à 3°C. Immédiatement, le mélange se décolore, et le précipité rougeâtre se dissout en quelques minutes.

Après agitation à 0°C pendant 1h, une analyse d'un échantillon par ¹H-RMN (D₂O) montre que l'adduit Herc-Cys est formé avec un rendement réactionnel de 55%.

Le bain de glace est enlevé, et on laisse agiter le mélange réactionnel pendant une heure. La température interne monte à 10°C. Le produit obtenu n'est pas isolé du milieu réactionnel et est utilisé directement dans l'étape suivante.

### b) Formation de la L-Ergothionéine

On ajoute ensuite 87,7 mL (106 g, 10 équiv.) d'acide 3-mercaptopropionique au mélange, et l'on chauffe sous forte agitation à 80°C pour 22h.

Une analyse d'un échantillon par ¹H-RMN (D₂O) montre que l'adduit Herc-Cys est complètement clivé en Ergothionéine.

### c) Isolement de la L-Ergothionéine

Après refroidissement à température ambiante, le mélange d'une couleur orange-marron est extrait avec 4x400 mL d'acétate d'éthyle.

La phase aqueuse est retenue, et le pH est ajusté à 4,5 - 5 avec une solution d'ammoniaque aqueuse à 20% (environ 21 mL). Afin de piéger l'excès de la L-cystéine présente dans le milieu, on ajoute 50,8 mL (53,0 g, 5 équiv.) de benzaldéhyde (selon M. P. Schubert, J. Biol. Chem. 114, 341-350 (1936*)* ou M. Seki et al. , J. Org. Chem. 67 (16), 5532 (2002))*.*

Le mélange est agité à température ambiante pendant 15h, et l'acide 2-phénylthiazolidine-4-carboxylique précipite sous forme d'un solide jaune claire. Après filtration du solide et rinçage avec 4x50 mL d'eau, le filtrat est extrait avec 2x200 mL d'acétate d'éthyle.

### d) Purification après désalinisation de la phase aqueuse avec une résine

Pour faciliter la cristallisation du produit final, la phase aqueuse retenue est désalinisée. Pour cela, elle est traitée par exemple avec de la résine Amberlite IRA 410 sous forme hydrogénocarbonate (selon K. A. Piez et al., J. Biol. Chem. 194, 669-672 (1952))*.* On ajoute 120 g de la résine au mélange réactionnel et on agite pendant 2h à température ambiante. Un fort dégagement gazeux est observé, ainsi qu'une décoloration progressive du milieu. Par ailleurs, le pH du mélange réactionnel descend vers pH = 8. Après 2h de temps de contact, on filtre la résine. Après rinçage avec 5x20 mL d'eau, l'opération est répétée encore 2 fois.

Le filtrat est ensuite évaporé à sec, et le solide obtenu est recristallisé avec de l'éthanol aqueux. On obtient 8,21g (34,9%) de L-Ergothionéine sous forme d'une poudre blanche.
¹H-RMN (D₂O, 400 MHz) : δ (ppm) = 3,20 (m, 2H) ; 3,29 (s, 9H) ; 3,90 (dd, J = 11 Hz, J = 5 Hz, 1H) ; 6, 81 (s, 1H).
UPLC-MS (ES+): 230,6 (NH+)
[α]_{D} = +124,6° (c=1, H₂O)

### Exemple 5 : Préparation de L-Ergothionéine à partir de l'Hercynine (« one-pot ») - purification après désalinisation de la phase aqueuse par électrodialyse

### a) Formation de l'adduit Herc-Cys (composé de formule (II))

98,6 g (0,5 mole) d'Hercynine sont dissous dans 1,5 L d'eau. La solution est transférée dans un réacteur en verre double enveloppe avec agitation mécanique. On ajoute 41,75 mL (0,5 mole) d'acide chlorhydrique concentré, puis on refroidit la solution à 0°C. Sous très forte agitation, 34 mL (106 g, 0,66 mole, 1,3 équiv.) de dibrome sont ajoutés goutte à goutte sans dépasser 2°C (temps d'addition 6,5min). Le mélange réactionnel se colore en jaune, et des flocons rougeâtres se forment. Sept minutes après la fin de l'addition du dibrome, 432 g (3,5 moles, 7 équiv.) de L-Cystéine sont ajoutés, et la température interne monte à 4°C. Immédiatement, le mélange se décolore, et le précipité rougeâtre se dissout en quelques minutes. On obtient une suspension blanchâtre.

Après agitation à 0°C pendant 1h, une analyse d'un échantillon par ¹H-RMN (D₂O) montre que l'adduit Herc-Cys est formé avec un rendement réactionnel de 56%.

Le système de refroidissement est arrêté, et on laisse agiter le mélange réactionnel pendant une heure. La température interne monte à 10°C.

### b) Formation de la L-Ergothionéine

On ajoute ensuite 441 mL (533 g, 5 moles, 10 équiv.) d'acide 3-mercaptopropionique au mélange, et l'on chauffe sous forte agitation à 80°C pour 24h.

Une analyse d'un échantillon par ¹H-RMN (D₂O) montre que l'adduit Herc-Cys est complètement clivé en Ergothionéine.

### c) Isolement de la L-Ergothionéine

Après refroidissement à température ambiante, le mélange d'une couleur orange-marron est extrait avec 4 x 2 L d'acétate d'éthyle.

La phase aqueuse est retenue, et le pH est ajusté à 4,5 - 5 avec une solution d'ammoniaque aqueuse à 20% (environ 110 mL). Afin de piéger l'excès de la cystéine présente dans le milieu, on ajoute 359 mL (375 g, 3,5 moles, 7 équiv.) de benzaldéhyde (selon M. P. Schubert, J. Biol. Chem. 114, 341-350 (1936*) ou* M. Seki et al. , J. Org. Chem. 67 (16), 5532 (2002*)*)*.*

Le mélange est agité à température ambiante pendant 15h, et la 2-phénylthiazolidine-4-carboxylique acide précipite sous forme d'un solide jaune clair. Après filtration du solide et rinçage avec 4x500 mL d'eau, le filtrat est extrait avec 4 x 1,5 L d'acétate d'éthyle.

### d) Purification après désalinisation de la phase aqueuse par électrodialyse

Pour faciliter la cristallisation du produit final, la phase aqueuse retenue est désalinisée. Pour cela, elle est par exemple désalinisée par électrodialyse (Bench Scale Electrodialysis Pump System BED 1-3 de PCCell (Allemagne), cellule ED200-020, 20 paires de membranes (cation échange PC-SK, anion-échange PC-SA), 10V).

La solution désalinisée est ensuite évaporée à sec, et le solide obtenu est recristallisé avec de l'éthanol aqueux. On obtient 47,68 g (41%) de L-Ergothionéine sous forme d'une poudre blanche.
¹H-RMN (D₂O, 400 MHz) : δ (ppm) = 3,20 (m, 2H) ; 3,29 (s, 9H) ; 3,90 (dd, J = 11 Hz, J = 5 Hz, 1H) ; 6, 81 (s, 1H).
UPLC-MS (ES+): 230,6 (MH+)
[α]_{D} = +125,2° (c=1, H₂O)

## Revendications

1. Procédé de synthèse d'un dérivé de formule (I) suivante : 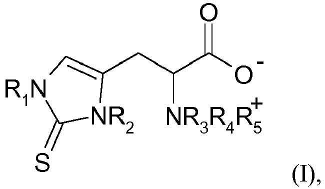 ou d'un sel physiologiquement acceptable de celui-ci, d'un tautomère, d'un stéréoisomère ou d'un mélange de stéréoisomères en toutes proportions,
pour laquelle :
- R₁ et R₂ représentent, indépendamment l'un de l'autre, un atome d'hydrogène ou un groupe (C₁-C₄)alkyle, au moins un des groupements R₁ et R₂ représentant un atome d'hydrogène, et
- R₃, R₄ et R₅ représentent, indépendamment les uns des autres, un groupe (C₁-C₄)alkyle,
comprenant les étapes successives suivantes :
(i) réaction de clivage d'un composé de type bétaïne de formule (II) suivante : 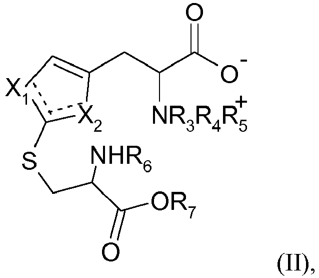 ou d'un sel physiologiquement acceptable, d'un tautomère, d'un stéréoisomère ou d'un mélange de stéréoisomères en toutes proportions, pour laquelle :
- 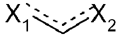 représente 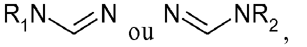
- R₁, R₂, R₃, R₄ et R₅ sont tels que définis ci-dessus,
- R₆ représente un atome d'hydrogène ou un groupe (C₁-C₄)alkyle ou -CO-((C₁-C₄)alkyle), et
- R₇ représente un atome d'hydrogène ou un groupe (C₁-C₄)alkyle, ,
en présence d'un thiol, à une température supérieure ou égale à 60°C, pour donner un composé de formule (I), et
(ii) séparation du composé de formule (I) obtenu à l'étape (i) précédente du milieu réactionnel.

2. Procédé selon la revendication 1, **caractérisé en ce que** le thiol répond à la formule R-SH, avec R représentant une chaîne alkyle, linéaire ou ramifiée, et de préférence linéaire, comportant de 1 à 8, notamment 2 à 6, atomes de carbone, substituée par un ou plusieurs groupes choisis parmi OH, SH, NH₂ et COOH.

3. Procédé selon la revendication 2, **caractérisé en ce que** le thiol est choisi parmi la cystéine, le dithiothréitol, le 2-mercaptoéthanol, l'acide 2-mercaptopropionique, l'acide 3-mercaptopropionique, l'acide mercaptoacétique, l'acide mercaptohexanoïque et l'acide thioglycolique, et de préférence est l'acide 3-mercaptopropionique.

4. Procédé selon la revendication 1, **caractérisé en ce que** l'étape (i) est réalisée à une température comprise entre 60 et 120°C, notamment entre 80 et 100°C.

5. Procédé selon l'une quelconque des revendications 1 à 4, **caractérisé en ce que** le composé de formule (I) répond à la formule (Ia) suivante : 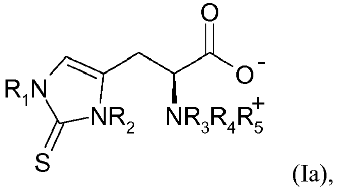 ou à un sel physiologiquement acceptable de celui-ci, à un tautomère, à un stéréoisomère ou à un mélange de stéréoisomères en toutes proportions, en particulier à un mélange d'énantiomères, et notamment à un mélange racémique de celui-ci,
pour laquelle R₁, R₂, R₃, R₄ et R₅ sont tels que définis à la revendication 1.

6. Procédé selon l'une quelconque des revendications 1 à 5, **caractérisé en ce que** le composé de formule (I) représente l'ergothionéine, et notamment la L-ergothionéine.

7. Procédé selon l'une quelconque des revendications 1 à 6, **caractérisé en ce que** le composé de type bétaine de formule (II) est préparé à partir d'un sel d'addition d'acide, à l'exclusion du sel de l'acide iodhydrique, d'un composé de type bétaïne de formule (III) suivante : 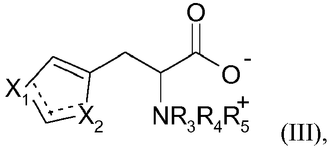 ou d'un tautomère, d'un stéréoisomère ou d'un mélange de stéréoisomères en toutes proportions, en particulier d'un mélange d'énantiomères, et notamment d'un mélange racémique de celui-ci,
pour laquelle 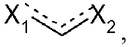 R₃, R₄ et R₅ sont tels que définis à la revendication 1, par réaction successivement avec du dibrome,
puis avec un dérivé de cystéine de formule (IV) suivante : 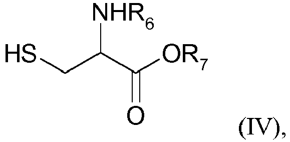 ou un stéréoisomère ou un mélange de stéréoisomères en toutes proportions, en particulier un mélange d'énantiomères, et notamment un mélange racémique de celui-ci,
dans laquelle R₆ et R₇ sont tels que définis à la revendication 1.

8. Procédé selon la revendication 7, **caractérisé en ce que** le composé de formule (I) répond à la formule (Ia) telle que définie à la revendication 5 et que le procédé comprend les étapes successives suivantes :
(a2) réaction d'un sel d'addition d'acide, à l'exclusion du sel de l'acide iodhydrique, d'un composé de type bétaïne de formule (IIIa) suivante : 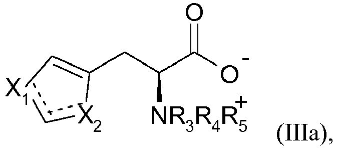 ou d'un tautomère, d'un stéréoisomère ou d'un mélange de stéréoisomères en toutes proportions, en particulier d'un mélange d'énantiomères, et notamment d'un mélange racémique de celui-ci,
pour laquelle 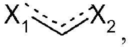 R₃, R₄ et R₅ sont tels que définis à la revendication 1, successivement avec du dibrome,
puis avec un dérivé de cystéine de formule (IV) tel que défini à la revendication 7 ou un stéréoisomère ou un mélange de stéréoisomères en toutes proportions, en particulier un mélange d'énantiomères, et notamment un mélange racémique de celui-ci, et en particulier avec de la cystéine et notamment de la L-cystéine
pour donner un composé de type bétaïne de formule (IIa) suivante : 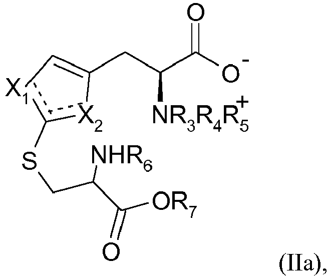 ou un sel physiologiquement acceptable, un tautomère, un stéréoisomère ou un mélange de stéréoisomères en toutes proportions, en particulier un mélange d'énantiomères, et notamment un mélange racémique de celui-ci,
pour laquelle 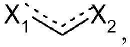 R₃, R₄, R₅, R₆ et R₇ sont tels que définis à la revendication 1,
(b2) réaction de clivage du composé de type bétaïne de formule (IIa) obtenu à l'étape (a2) précédente en présence d'un thiol, à une température supérieure ou égale à 60°C,
pour donner un composé de formule (Ia), et
(c2) séparation du composé de formule (Ia) obtenu à l'étape (b2) précédente du milieu réactionnel.

9. Procédé selon l'une quelconque des revendications 7 et 8, **caractérisé en ce que** le dérivé de cystéine est utilisé en excès, à raison de 2 à 10, avantageusement 3 à 7 équivalents molaires de dérivé de cystéine par rapport au composé de type bétaïne de formule (III).

10. Procédé selon l'une quelconque des revendications 7 à 9, **caractérisé en ce que** le dibrome est utilisé à raison de 1 à 1,5 équivalents molaires par rapport au composé de type bétaïne de formule (III).

11. Procédé selon l'une quelconque des revendications 7 à 10, **caractérisé en ce que** la préparation du composé (I) à partir du composé (III) est réalisé dans un seul réacteur, sans isolation du composé (II) intermédiaire.

12. Utilisation d'un composé de type bétaïne de formule (II) suivante : 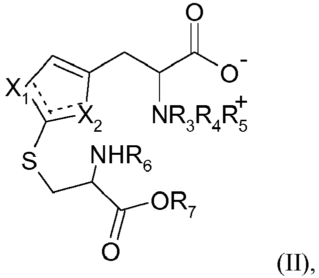 ou un sel physiologiquement acceptable, un tautomère, un stéréoisomère ou un mélange de stéréoisomères en toutes proportions, en particulier un mélange d'énantiomères, et notamment un mélange racémique de celui-ci,
pour laquelle :
- 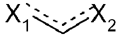 représente 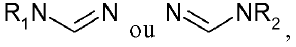 et
- R₁, R₂, R₃, R₄, R₅, R₆ et R₇ sont tels que définis à la revendication 1,
a l' exclusion du composé pour lequel 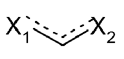 représente 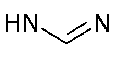 ou 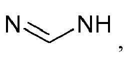 , R₃, R₄ et R₅ représentent chacun un groupe méthyle et R₆ et R₇ représentent chacun un atome d'hydrogène
pour la préparation d'un dérivé de formule (I) suivante : 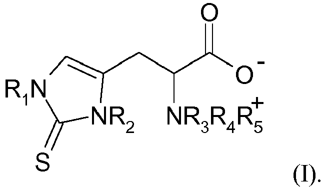 par synthèse chimique comprenant le clivage en présence d'un thiol par chauffage à une température supérieure ou égale à 60°C.

## Patentansprüche

1. Verfahren zur Synthese eines Derivats der folgenden Formel (I): 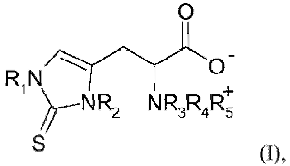 oder eines physiologisch verträglichen Salzes davon, eines Tautomers, eines Stereoisomers oder eines Gemischs von Stereoisomeren in allen Verhältnissen,
wobei:
- R₁ und R₂ unabhängig voneinander ein Wasserstoffatom oder eine Gruppe (C₁-C₄)-Alkyl darstellen, wobei mindestens eine der Gruppen R₁ und R₂ ein Wasserstoffatom darstellt, und
- R₃, R₄ und R₅ unabhängig voneinander eine Gruppe (C₁-C₄)-Alkyl darstellen,
welche die nachstehenden aufeinanderfolgenden Schritte umfasst:
(i) Spaltungsreaktion einer Betainverbindung der folgenden Formel (II): 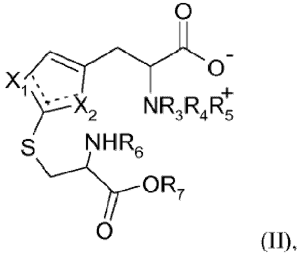 oder eines physiologisch verträglichen Salzes davon, eines Tautomers, eines Stereoisomers oder eines Gemischs von Stereoisomeren in allen Verhältnissen, wobei
- 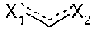 für 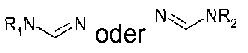 steht,
- R₁, R₂, R₃, R₄ und R₅ wie vorstehend definiert sind,
- R₆ ein Wasserstoffatom oder eine Gruppe (C₁-C₄)-Alkyl oder CO-((C₁-C₄)Alkyl) darstellt, und
- R₇ ein Wasserstoffatom oder eine Gruppe (C₁-C₄)-Alkyl darstellt, in Gegenwart eines Thiols bei einer Temperatur höher oder gleich 60°C, um eine Verbindung der Formel (I) zu ergeben, und (ii)Abtrennung der im vorangehenden Schritt (i) erhaltenen Verbindung der Formel (I) vom Reaktionsmedium.

2. Verfahren nach Anspruch 1, **dadurch gekennzeichnet, dass** das Thiol der Formel R-SH entspricht, mit R, das eine lineare oder verzweigte Alkylkette darstellt, vorzugsweise linear, mit 1 bis 8, ganz besonders mit 2 bis 6 Kohlenstoffatomen, substituiert mit einer oder mehreren Gruppen ausgewählt aus OH, SH, NH₂ und COOH.

3. Verfahren nach Anspruch 2, **dadurch gekennzeichnet, dass** das Thiol ausgewählt ist aus Cystein, Dithiothreitol, 2-Mercaptoethanol, 2-Mercapto-propionsäure, 3-Mercaptopropionsäure, Mercaptoessigsäure, Mercapto-hexansäure und Thioglykolsäure und vorzugsweise 3-Mercaptopropionsäure ist.

4. Verfahren nach Anspruch 1, **dadurch gekennzeichnet, dass** Schritt (i) bei einer Temperatur zwischen 60 und 120°C, ganz besonders zwischen 80 und 100°C, erfolgt.

5. Verfahren nach einem der Ansprüche 1 bis 4, **dadurch gekennzeichnet, dass** die Verbindung der Formel (I) der folgenden Formel (la) entspricht: 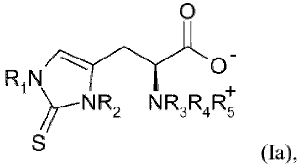 oder einem physiologisch verträglichen Salz davon, einem Tautomer, einem Stereoisomer oder einem Gemisch aus Stereoisomeren in allen Verhältnissen, insbesondere einem Gemisch aus Enantiomeren und ganz besonders einem racemischen Gemisch davon, wobei R₁, R₂, R₃, R₄ und R₅ wie in Anspruch 1 definiert sind.

6. Verfahren nach einem der Ansprüche 1 bis 5, **dadurch gekennzeichnet, dass** die Verbindung der Formel (I) Ergothionein und insbesondere L-Ergothionein darstellt.

7. Verfahren nach einem der Ansprüche 1 bis 6, **dadurch gekennzeichnet, dass** die Betainverbindung der Formel **(II)** aus einem Säureadditionssalz, davon ausgeschlossen das Salz von Jodwasserstoffsäure, einer Betainverbindung der folgenden Formel (III) hergestellt wird: 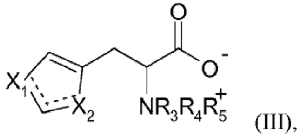 oder aus einem Tautomer, einem Stereoisomer oder einem Gemisch aus Stereoisomeren in allen Verhältnissen, insbesondere einem Gemisch aus Enantiomeren und ganz besonders aus einem racemischen Gemisch davon,
wobei 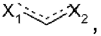 R₃, R₄ und R₅ wie in Anspruch 1 definiert sind, durch aufeinanderfolgende Reaktion mit Dibrom,
dann mit einem Cystein-Derivat der folgenden Formel (IV): 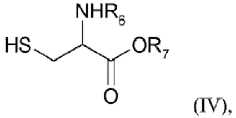 oder einem Stereoisomer oder einem Gemisch aus Stereoisomeren in allen Verhältnissen, insbesondere einem Gemisch aus Enantiomeren und ganz besonders aus einem racemischen Gemisch davon, wobei R₆ und R₇ wie in Anspruch 1 definiert sind.

8. Verfahren nach Anspruch 7, **dadurch gekennzeichnet, dass** die Verbindung der Formel (I) der Formel (la) wie in Anspruch 5 definiert entspricht und, dass das Verfahren die nachstehenden aufeinanderfolgenden Schritte umfasst:
(a2) Reaktion eines Säureadditionssalzes, davon ausgeschlossen das Salz von Jodwasserstoffsäure, von einer Betainverbindung der folgenden Formel (IIIa): 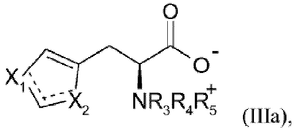 oder einem Tautomer, einem Stereoisomer oder einem Gemisch aus Stereoisomeren in allen Verhältnissen, insbesondere einem Gemisch aus Enantiomeren und ganz besonders aus einem racemischen Gemisch davon,
wobei, 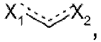 R₃ R₄ und R₅ wie in Anspruch 1 definiert sind, aufeinanderfolgend mit Dibrom,
dann mit einem Cystein-Derivat der Formel (IV) wie in Anspruch 7 definiert oder einem Stereoisomer oder einem Gemisch aus Stereoisomeren in allen Verhältnissen, insbesondere einem Gemisch aus Enantiomeren und ganz besonders aus einem racemischen Gemisch davon und insbesondere mit Cystein und ganz besonders mit L-Cystein,
um eine Betainverbindung der folgenden Formel (IIa) zu ergeben: 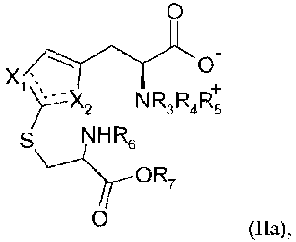 oder ein physiologisch verträgliches Salz, ein Tautomer, ein Stereoisomer oder ein Gemisch aus Stereoisomeren in allen Verhältnissen, insbesondere ein Gemisch aus Enantiomeren und ganz besonders ein racemisches Gemisch davon,
wobei 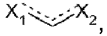 R₃, R₄, R₅, R₆ und R₇ wie in Anspruch 1 definiert sind,
(b2) Spaltungsreaktion der im vorangehenden Schritt (a2) erhaltenen Betainverbindung der Formel (IIa) in Gegenwart eines Thiols bei einer Temperatur höher oder gleich 60°C, um eine Verbindung der Formel (la) zu ergeben, und
(c2) Abtrennung der im vorangehenden Schritt (b2) erhaltenen Verbindung der Formel (la) vom Reaktionsmedium.

9. Verfahren nach einem der Ansprüche 7 und 8, **dadurch gekennzeichnet, dass** in Bezug auf die Betainverbindung der Formel (III) das Cystein-Derivat im Überschuss in einer Menge von 2 bis 10, vorteilhafterweise mit 3 bis 7 Moläquivalenten von Cystein-Derivat verwendet wird.

10. Verfahren nach einem der Ansprüche 7 bis 9, **dadurch gekennzeichnet, dass** in Bezug auf die Betainverbindung der Formel (III) das Dibrom in einer Menge von 1 bis 1,5 Moläquivalenten verwendet wird.

11. Verfahren nach einem der Ansprüche 7 bis 10, **dadurch gekennzeichnet, dass** die Herstellung der Verbindung (I) ausgehend von der Verbindung (III) in einem einzigen Reaktor ohne Isolierung der Zwischenverbindung **(II)** erfolgt.

12. Verwendung einer Betainverbindung der folgenden Formel (II): 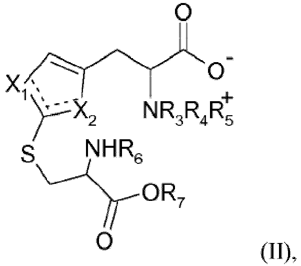 oder eines physiologisch verträglichen Salzes, eines Tautomers, eines Stereoisomers oder eines Gemischs von Stereoisomeren in allen Verhältnissen, insbesondere eines Gemischs aus Enantiomeren und ganz besonders eines racemischen Gemischs davon,
wobei:
- 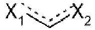 für 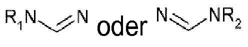 steht, und
- R₁, R₂, R₃, R₄, R₅, R₆ und R₇ wie in Anspruch 1 definiert sind,
davon ausgeschlossen die Verbindung, für die 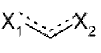 für 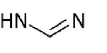 oder 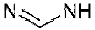 steht, wobei R₃, R₄ und R₅ jeweils eine Methylgruppe darstellen und R₆ und R₇ jeweils ein Wasserstoffatom zur Herstellung eines Derivats der folgenden Formel (I) darstellen: 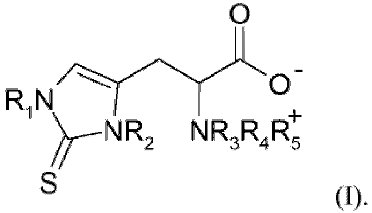 durch chemische Synthese, die eine Spaltung in Gegenwart eines Thiols durch Erwärmen auf eine Temperatur von höher oder gleich 60°C umfasst.

## Claims

1. Method of synthesizing a derivative of the following formula (I): 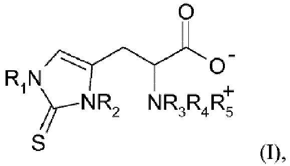 or a physiologically acceptable salt thereof, a tautomer, a stereomer or a mixture of stereomers in any proportion,
wherein:
- R₁ and R₂ represent, independently of each other, a hydrogen atom or a (C₁-C₄) alkyl group, at least one of the R₁ and R₂ groups representing a hydrogen atom, and
- R₃, R₄ and R₅ represent, independently of each other, a (C₁-C₄) alkyl group,
comprising the following successive steps:
(i) cleavage reaction of a betaine type compound of the following formula **(II)** 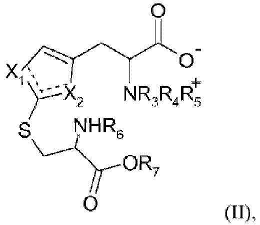 or a physiologically acceptable salt, a tautomer, a stereomer or a mixture of stereomers in any proportion, wherein:
- 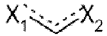 represents 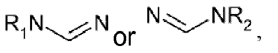
- R₁, R₂, R₃, R₄ and R₅ are as defined above,
- R₆ represents a hydrogen atom or a (C₁-C₄) alkyl or -CO-((C₁-C₄) alkyl group, and
- R₇ represents a hydrogen atom or a (C₁-C₄) alkyl group,
in the presence of a thiol, at a temperature greater than or equal to 60°C, to give a compound of formula (I), and
(ii) separation of a compound of formula (I) obtained in the preceding step
(i) from the reaction medium.

2. Method according to claim 1, **characterized in that** the thiol has the formula R-SH, with R representing a linear or branched alkyl chain, preferably linear, comprising from 1 to 8, in particular 2 to 6, carbon atoms, substituted by one or more groups chosen from OH, SH, NH₂ and COOH.

3. Method according to claim 2, **characterized in that** the thiol is chosen from cysteine, dithiothreitol, 2-mercaptoethanol, 2-mercaptopropionic acid, 3-mercaptopropionic acid, mercaptoacetic acid, mercaptohexanoic acid and thioglycolic acid, and preferably is 3-mercaptoproionic acid.

4. Method according to claim 1, **characterized in that** step (i) is performed at a temperature of between 60 and 120°C, in particular between 80 and 100°C.

5. Method according to any of claims 1 to 4, **characterized in that** the compound of formula (I) has the following formula (Ia): 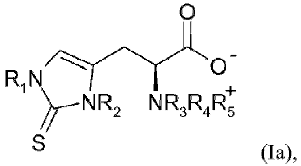 or a physiologically acceptable salt thereof, a tautomer, a stereomer or a mixture of stereomers in any proportion, in particular a mixture of enantiomers, and in particular a racemic mixture thereof,
wherein R₁, R₂, R₃, R₄ and R₅ are as defined in claim 1.

6. Method according to any of claims 1 to 5, **characterized in that** the compound of formula (I) represents ergothioneine, and in particular L-ergothioneine.

7. Method according to any of claims 1 to 6, **characterized in that** the betaine type compound of formula **(II)** is prepared from an acid addition salt, excluding hydriodic acid salt, of a betaine type compound of the following formula (III): 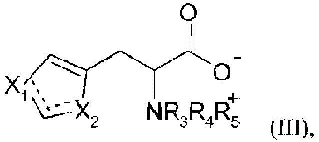 or a tautomer, a stereomer or a mixture of stereomers in any proportion, in particular a mixture of enantiomers, and in particular a racemic mixture thereof,
wherein 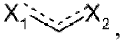 R₃, R₄ and R₅ are as defined in claim 1, successively by reaction with bromine,
then with a cysteine derivative of the following formula (IV): 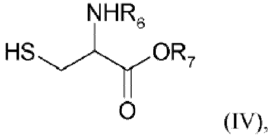 or a stereomer or a mixture of stereomers in any proportion, in particular a mixture of enantiomers, and in particular a racemic mixture thereof,
wherein R₆ and R₇ are as defined in claim 1.

8. Method according to claim 7, **characterized in that** the compound of formula (I) has the formula (Ia) as defined in claim 5 and the method comprises the following successive steps:
(a2) reaction of an acid addition salt, excluding hydriodic acid salt, of a betaine type compound of the following formula (IIIa): 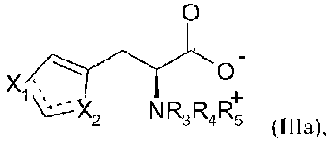 or a tautomer, a stereomer or a mixture of stereomers in any proportion, in particular a mixture of enantiomers, and in particular a racemic mixture thereof,
wherein 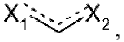 R₃, R₄ and R₅ are as defined in claim 1, successively with bromine,
then with a cysteine derivative of formula (IV) as defined in claim 7 or a stereomer or a mixture of stereomers in any proportion, in particular a mixture of enantiomers, and in particular a racemic mixture thereof, and in particular with cysteine and in particular L-cysteine
to give a betaine type compound of the following formula (IIa): 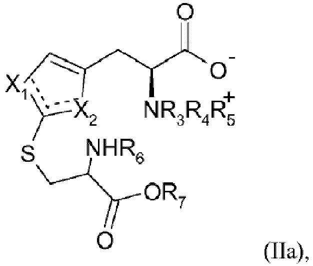 or a physiologically acceptable salt, a tautomer, a stereomer or a mixture of stereomers in any proportion, in particular a mixture of enantiomers, and in particular a racemic mixture thereof,
wherein 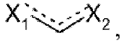 R₃, R₄, R₅, R₆ and R₇ are as defined in claim 1,
(b2) cleavage reaction of a betaine type compound of formula (IIa) obtained in the preceding step (a2) in the presence of a thiol, at a temperature greater than or equal to 60°C, to give a compound of formula (Ia), and
(c2) separation of the compound of formula (Ia) obtained in the preceding step (b2) from the reaction medium.

9. Method according to any of claims 7 and 8, **characterized in that** the cysteine derivative is used in excess, in a ratio of 2 to 10, advantageously 3 to 7 molar equivalents of cysteine derivative in relation to the betaine type compound of formula (III).

10. Method according to any of claims 7 to 9, **characterized in that** bromine is used in a ratio of 1 to 1.5 molar equivalents in relation to the betaine type compound of formula (III).

11. Method according to any of claims 7 to 10, **characterized in that** the preparation of compound (I) from compound (III) is achieved in a single reactor, without isolating of the intermediate compound (II).

12. Use of a betaine type compound of the following formula (II): 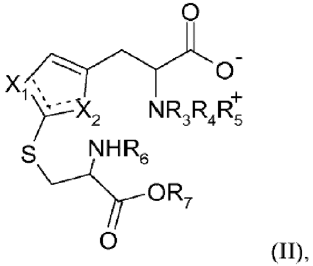 or a physiologically acceptable salt, a tautomer, a stereomer or a mixture of stereomers in any proportion, in particular a mixture of enantiomers, and in particular a racemic mixture thereof,
wherein:
- 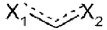 represents 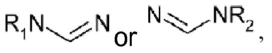 and
- R₁, R₂, R₃, R₄, R₅, R₆ and R₇ are defined in claim 1.
excluding the compound for which 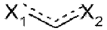 represents 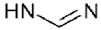 or 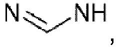 R₃, R₄ and R₅ each represent a methyl group and R₆ and R₇ each represent a hydrogen atom
for the preparation of a derivative of the following formula (I): 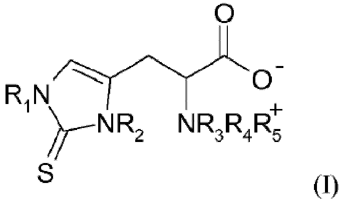 by chemical synthesis comprising cleavage in the presence of a thiol by heating to a temperature greater than or equal to 60°C.
